# EUROPEAN PATENT SPECIFICATION

(11) **EP 2 623 141 B1**
(45) Date of publication and mention of the grant of the patent: **27.10.2021**
(21) Application number: 11828330.8
(22) Date of filing: 12.09.2011
(51) Int. Cl.: G16H 20/17, G16H 40/63

(54) **MEDICAL PUMP HOUSING RACK AND CONTROL METHOD THEREOF**
TRÄGER FÜR EIN MEDIZINISCHES PUMPENGEHÄUSE UND VERFAHREN ZU SEINER STEUERUNG
SUPPORT DE BOÎTIER DE POMPE MÉDICALE ET PROCÉDÉ DE COMMANDE DE CELUI-CI

(30) Priority: 29.09.2010 JP 2010218978
(43) Date of publication of application: 07.08.2013
(73) Proprietor: Terumo Kabushiki Kaisha, Tokyo 151-0072 (JP)
(72) Inventor: HORIGUCHI, Hiroko, Ashigarakami-gun Kanagawa 259-0151 (JP); FUJII, Toshihiro, Ashigarakami-gun Kanagawa 259-0151 (JP)
(74) Representative: Fédit-Loriot
(86) International application number: PCT/JP2011/005109
(87) International publication number: WO 2012/042763

(56) References cited:
- JP-A- 1 265 973
- JP-A- 2004 097 757
- JP-A- 2006 500 077
- JP-A- 2008 532 635
- US-A- 4 925 444
- US-A1- 2005 085 760
- US-A1- 2009 177 188
- None

## Description

### TECHNICAL FIELD

The present invention relates to a medical pump housing rack which houses a plurality of arranged medical pumps and a control method therefor.

### BACKGROUND ART

In medical settings such as a hospital, as a pump for administering a drug into the body of a patient, there is used an infusion pump which injects a drug in an infusion bag into the body through an infusion tube at a controlled infusion speed, a syringe pump which injects a drug in a syringe into the body at a controlled infusion speed, or the like.

In general, such an infusion pump and a syringe pump (these pumps will be collectively termed medical pumps in this specification) are used to infuse one type of drug. For this reason, in order to simultaneously administer a plurality of types of drugs into a patient in an operating room, intensive care unit, or the like, it is necessary to use a plurality of medical pumps corresponding to the types of drugs.

In addition, an infusion bag or syringe is charged with only a predetermined amount of drug in advance. To administer a large amount of the same type of drug into a patient, it is necessary to prepare a plurality of medical pumps corresponding to the amount to be administered in advance and sequentially operate them (note that a method of continuously administering the same type of drug by sequentially operating a plurality of medical pumps in this manner is called the "W method").

As described above, in many medical fields, a plurality of medical pumps are used for one patient. In such a case, juxtaposing medical pumps on a desk or placing them on a floor will interfere with operation by a doctor or the like. In addition, moving these pumps will increase the operation load. The common practice is therefore to use a medical pump housing rack which houses arranged medical pumps (see, for example, PTL1).

### CITATION LIST

### PATENT LITERATURE

PTL1: Japanese Patent Laid-Open No. 11-347118

Document US20090177188 represents the closest prior art and discloses a medical pump housing rack with control unit, communication module and a plurality of medical pumps, wherein the plurality of pumps is connected to the control unit by a data connection network and not by a plurality of numbered ports of the rack, and wherein the control unit determines cooperation permission based on matching drug information and not based on the pumps' adjacent housing, and wherein the medical pumps are interconnected in star-shape or in series but not interconnected via said communication module.

### SUMMARY OF INVENTION

### TECHNICAL PROBLEM

If, however, a plurality of medical pumps are arranged and housed in a medical pump housing rack, there is a risk of operating a medical pump different from a medical pump to be operated. When operating a plurality of medical pumps in cooperation with each other by the W method described above, the setting operation, the operation instruction operation, and the like become more complicated than those in a general infusing operation. This tends to cause operation errors.

When performing infusion by the W method, it is important for each of the medical pumps to be operated in cooperation with each other to monitor not only an operation error on itself by the user but also the operation states of other medical pumps to be operated in cooperation. This is because it is necessary to cancel the cooperation or stop operation depending on the operation states of other medical pumps.

For the above reason, when performing infusion by the W method using a plurality of medical pumps housed in a medical pump housing rack, the medical pump housing rack is preferably configured to prevent the user from making operation errors by monitoring each medical pump.

The present invention has been made in consideration of the above problem, and has as its object to provide a medical pump housing rack which monitors a plurality of medical pumps which perform infusion by the W method and reduces operation errors on the medical pumps by the user.

### SOLUTION TO PROBLEM

In order to achieve the above object, a medical pump housing rack according to the present invention is defined in claims 1-5, a control method for a medical pump housing rack is defined in claim 6, and a computer program for causing a computer to execute said control method is defined in claim 7.

### ADVANTAGEOUS EFFECTS OF INVENTION

According to the present invention, it is possible to provide a medical pump housing rack which monitors a plurality of medical pumps which perform infusion by the W method and reduces operation errors on the medical pumps by the user.

Other features and advantages of the present invention will be apparent from the following description taken in conjunction with the accompanying drawings. Note that the same reference numerals denote the same or like components throughout the accompanying drawings.

### BRIEF DESCRIPTION OF DRAWINGS

The accompanying drawings are included in the specification, constitute a part of the specification, illustrate the embodiments of the present invention, and are used to explain the principle of the present invention together with the description.
Fig. 1 is a chart for explaining infusion by the W method;
Fig. 2A is a perspective view showing the outer arrangement of a medical pump housing rack;
Fig. 2B is a perspective view showing the outer arrangement of the medical pump housing rack;
Fig. 3 is a block diagram showing a control arrangement in a medical pump housing rack housing a plurality of medical pumps;
Fig. 4 is a view for explaining the operations of a starting pump and subsequent pump when performing infusion by the W method and various types of processes in the W method monitoring function of a medical pump housing rack 200 in this case;
Fig. 5 is a flowchart showing a detailed procedure for cooperation permission/inhibition determination processing in the medical pump housing rack;
Fig. 6 is a flowchart showing a detailed procedure for cooperation establishment determination processing in the medical pump housing rack; and
Fig. 7 is a flowchart showing a procedure for infusion start processing in a medical pump.

### DESCRIPTION OF EMBODIMENTS

Each embodiment of the present invention will be described in detail below with reference to the accompanying drawings, as needed. Note that the present invention is not limited to the following embodiments and can be modified, as needed.

However, the scope of the invention is defined only by the claims. The following embodiments merely define examples, and only those falling within the scope of the claims are part of the invention. Any subject-matter described as embodiments which does not fall within the scope of the claims are to be considered as illustrative only.

### <1. Explanation about Infusion by W Method>

Infusion by the W method will be described first. The W method is an administration method of continuously infusing the same drug to a patient without changing the flow rate by sequentially operating two medical pumps.

In general, in the operation of two medical pumps, if the flow rate of infusion by each medical pump is low, the startup of the second medical pump (subsequent pump) for infusion slows down. The W method is designed to avoid variations in flow rate accompanying such an infusion startup delay by starting infusion by the second pump (subsequent pump) a little before the end of infusion by the first pump (starting pump) instead of after the end of infusion by the first pump.

The W method controls infusion so as to keep the combined flow rate of the two medical pumps almost constant and set the flow rate of the subsequent pump to the flow rate at the cooperation start time when infusion by the starting pump is finally complete. Note that the manner of controlling the combined flow rate is not limited to this. For example, in order to avoid flow rate variations accompanying startup delays in infusion by the subsequent pump, the combined flow rate of the two medical pumps in cooperation may be controlled based on a reference flow rate which is set in advance such that the combined flow rate exceeds it (note that the "reference flow rate" is the flow rate of the starting pump immediately before cooperation start (the flow rate to be inherited by the subsequent pump)).

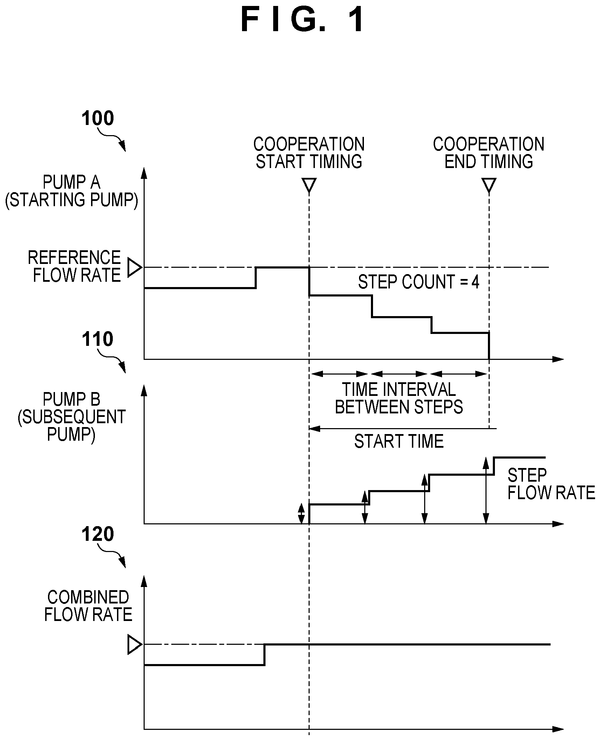

Fig. 1 is a chart showing temporal changes in the flow rate of each of two predetermined medical pumps (pumps A and B) and temporal changes in the combined flow rate of the two pumps when infusion is performed by the W method using the two pumps.

In this chart, a graph 100 represents temporal changes in the flow rate of the pump A (starting pump) which performs infusion first, and a graph 110 represents temporal changes in the flow rate of the pump B (subsequent pump) which performs infusion after the pump A.

Note that in the graphs 100 and 110, the operation of changing the flow rate stepwise will be referred to as a "step", and the number of times of changing the flow rate will be referred to as a "step count". In the case of the graphs 100 and 110, the step count is "4".

Assume that the step count, the time interval between the respective steps, and the flow rate in each step (step flow rate) are stored in advance in the form of a W method table in each medical pump in correspondence with drug information. When the user selects a drug for each medical pump and reads out a corresponding W method table, each medical pump sets a step count, the time interval between the respective steps, and a step flow rate in accordance with the read W method table.

In addition, setting a step count, the time interval between the respective steps, and a step flow rate will determine a change start timing (cooperation start timing) in changing the flow rate stepwise and a timing at which the flow rate becomes 0 (cooperation end timing). Assume that in this embodiment, the above step flow rate is set as a ratio when the flow rate of the pump A (starting pump) at the cooperation start timing is 100%.

Performing infusion by the W method will control the pumps A and B in accordance with the temporal changes indicated by the graphs 100 and 110. This keeps the combined flow rate of the pumps A and B almost constant and makes the flow rate of the pump B equal to the flow rate of pump A at the cooperation start timing when infusion by the pump A ends (at the cooperation end timing) (see a graph 120). Note that if, for example, the remaining amount of the pump A becomes 0 (or a remaining amount warning is issued to notify that the remaining amount of the pump A has become small) before transition to the last step in the W method table, control is performed to change the flow rate of pump B to the flow rate in the last step (the flow rate of the pump A at the cooperation start time) regardless of the W method table, thereby keeping the flow rate of the pump B equal to that of the pump A.

### <2. Outer Arrangement of Medical Pump Housing Rack>

The outer arrangement of the medical pump housing rack housing medical pumps which perform infusion by the W method will be described next.

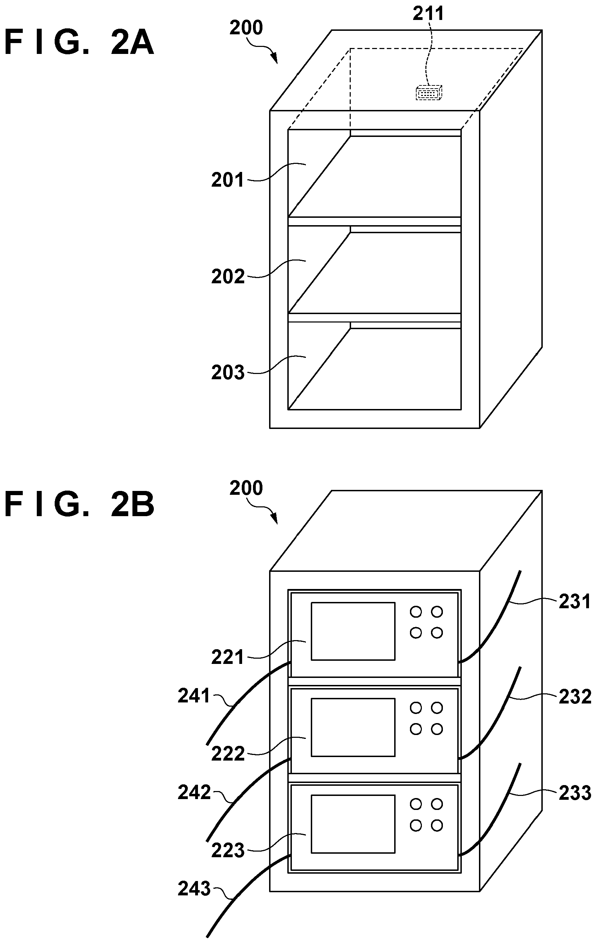

Fig. 2A is a perspective view showing the outer arrangement of a medical pump housing rack 200 housing a plurality of medical pumps. As shown in Fig. 2A, the medical pump housing rack 200 is provided with housing portions 201 to 203, each for housing one medical pump, in the vertical direction to vertically dispose a plurality of medical pumps (three medical pumps in this case).

The housing portions 201 to 203 open at the front to allow medical pumps to be inserted from the front surface side. The back surface sides of the housing portions 201 to 203 are provided with ports 211 to 213 (Fig. 2A shows only the port 211) in correspondence with the housing positions of medical pumps. Each of the ports 211 to 213 is formed from a connector which can be connected to a medical pump. The respective medical pumps are communicatively connected to the medical pump housing rack 200 via the ports 211 to 213. Assume that the ports 211 to 213 are respectively assigned with identification numbers #1, #2, and #3, beginning at the top, in the order of the housing positions of the pumps which are vertically disposed.

Fig. 2B is a perspective view showing the outer arrangement of the medical pump housing rack 200 housing a plurality of medical pumps.

Referring to Fig. 2B, medical pumps (infusion pumps) 221 to 223 infuse drugs in infusion bags (not shown) connected to tubes 231 to 233 on the right side on the drawing surface into a patient (not shown) connected to tubes 241 to 243 on the left side on the drawing surface.

The front surface sides of the medical pumps 221 to 223 are provided with operation buttons for inputting various kinds of settings and various kinds of operation instructions and display units which display infusion states and the like in the medical pumps 221 to 223. The user administers drugs into a patient by operating the respective operation buttons while watching the information displayed on the display units.

Note that according to the above description, the medical pump housing rack 200 houses three medical pumps. However, the present invention is not limited to this. The above description has exemplified the infusion pumps as the medical pumps to be housed in the medical pump housing rack 200. However, the present invention is not limited to this, and other medical pumps such as syringe pumps may be housed.

### <3. Control Arrangement in Medical Pump System>

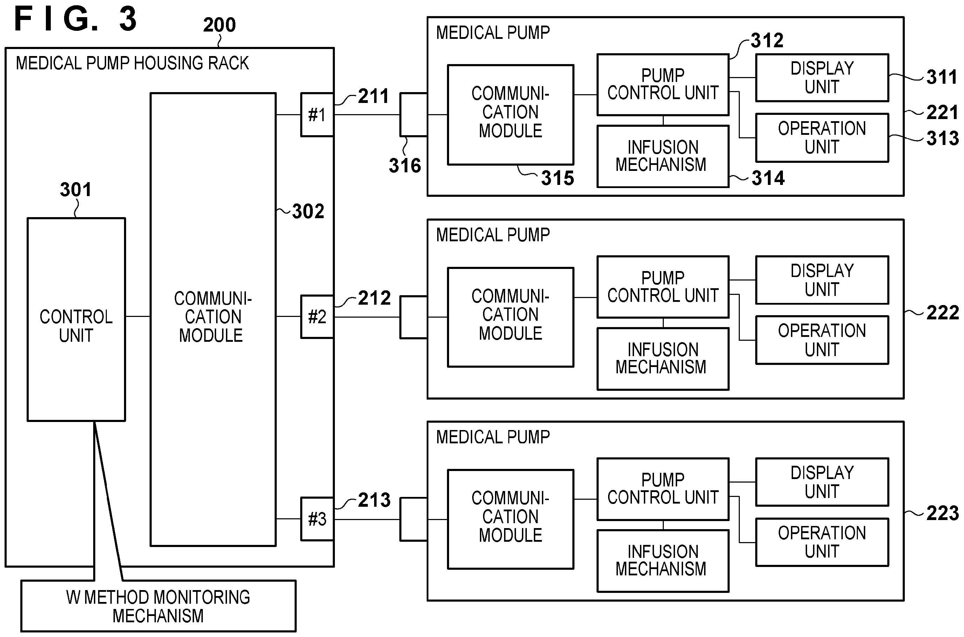

Fig. 3 is a block diagram showing an example of a control arrangement for the medical pump housing rack 200 and the medical pumps 221 to 223 according to this embodiment. The three ports 211 to 213 of the medical pump housing rack 200 are connected to a communication module 302. Upon receiving a signal (data) from one of the medical pumps 221 to 223 via a corresponding one of the ports 211 to 213, the communication module 302 notifies a control unit 301 of the received data and the received port number (one of the port numbers #1 to #3) .

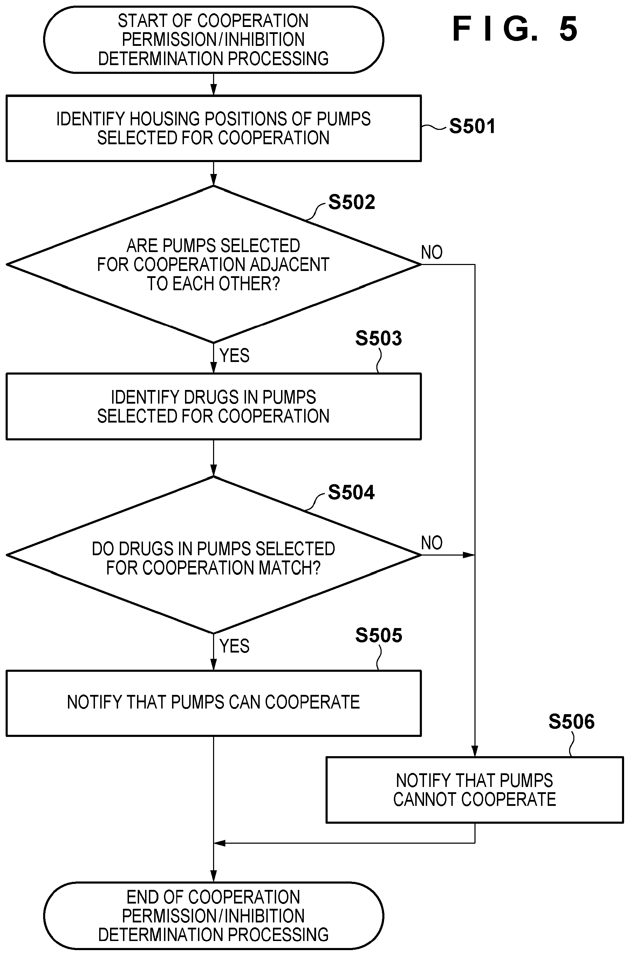

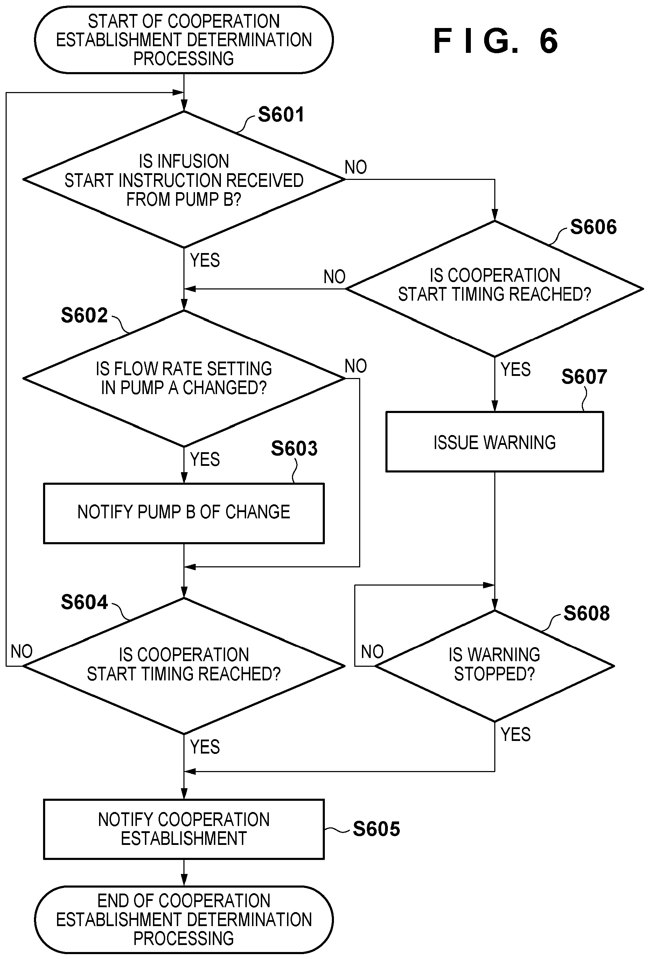

Upon receiving a pair of a port number and data (a command or the like) from the control unit 301, the communication module 302 outputs the data received from the control unit 301 to one of the ports 211 to 213 which corresponds to the port number. This allows the control unit 301 of the medical pump housing rack 200 to separately communicate with the plurality of housed medical pumps 221 to 223. Note that the control unit 301 is a computer including a CPU, ROM, and RAM (none of which are shown), and executes various types of processing programs concerning a W method monitoring function including processing (cooperation permission/inhibition determination processing and cooperation establishment determination processing) shown in the flowcharts of Figs. 5 and 6.

A display unit 311 of the medical pump 221 includes, for example, a liquid crystal panel, and performs various types of display operations under the control of a pump control unit 312. An operation unit 313 includes operation buttons for inputting various settings and various operation instructions.

A connector 316 is connected to the port 211 and implements electrical connection (communication) between the medical pump 221 and the medical pump housing rack 200. Assume that the connector 316 is connected to the port 211 via a cable. Alternatively, the connector 316 may be directly connected to the port 211 (for example, the connector 316 may be connected to the port 211 when the medical pump 221 is housed in the medical pump housing rack 200).

The pump control unit 312 controls an infusion mechanism 314 to control infusion. If the medical pump 221 is an infusion pump, the infusion mechanism 314 includes, for example, a plurality of fingers, which sequentially press an infusion tube to send out a drug into the tube. If the medical pump 221 is a syringe pump, the infusion mechanism 314 is attached with a syringe whose plunger is to be pressed.

The signal (data) input by the operation unit 313 is transmitted to the medical pump housing rack 200 via the pump control unit 312 and a communication module 315, and is used for various types of processes concerning the above W method monitoring function. The signal (data) transmitted by the medical pump housing rack 200 is received via the communication module 315, and is used by the pump control unit 312 for processing for infusion by the W method.

Described below is the W method monitoring function of the medical pump housing rack 200 in a case in which infusion by the W method is performed by using the medical pump 221 as the starting pump and the medical pump 222 as the subsequent pump in the control arrangement described above. Note that in the following description, the starting pump and the subsequent pump will be referred to as the "pump A" and the "pump B", respectively.

### <4. Operation at Time of Infusion by W Method>

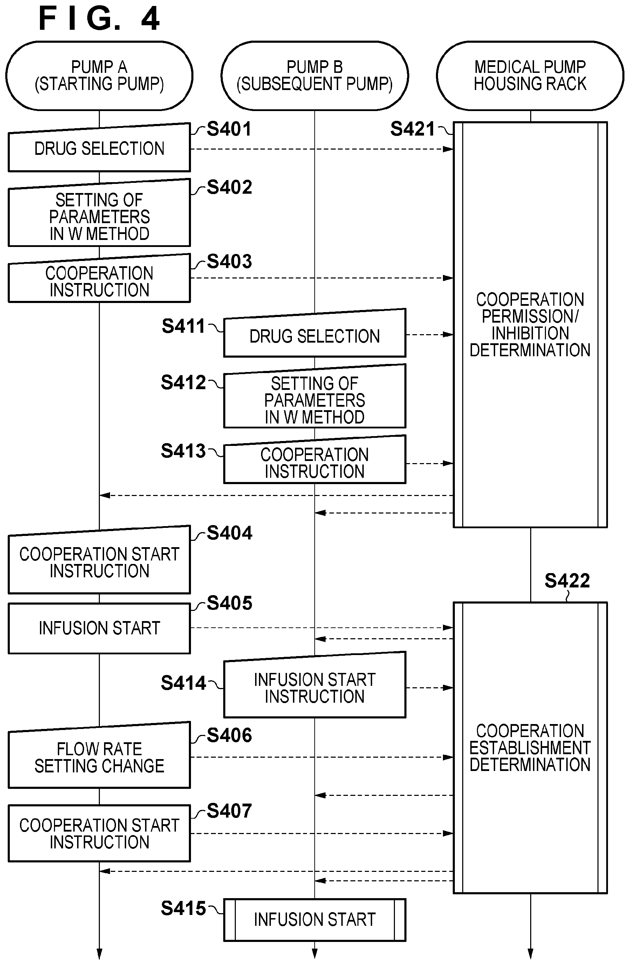

Fig. 4 is a chart for explaining the operations of the pump A (starting pump) and pump B (subsequent pump) in infusion by the W method and various types of processes by the W method monitoring function of the medical pump housing rack 200 in this case.

A method of deciding a starting pump and a subsequent pump will be described first. Assume that the medical pump housing rack 200 has received W method cooperation reservation notifications (W method available drug selection notifications) from the two medical pumps. In this case, if one medical pump has already performed infusion, the medical pump housing rack 200 recognizes the pump as the starting pump (pump A), and notifies the other medical pump that it is the subsequent pump (pump B). If the two medical pumps have not performed infusion, the medical pump housing rack 200 recognizes, as the starting pump, a pump from which an infusion start notification is received first after the establishment of cooperation reservation, and notifies the other medical pump that it is the subsequent pump. If the two medical pumps have already performed infusion, the medical pump housing rack 200 notifies the two pumps that cooperation is not established.

After the pumps A and B are decided, the pump A accepts the selection of a drug to be administered in step S401.

In step S402, the pump A reads out a W method table stored in correspondence with the drug accepted in step S401, and sets a step count, the time interval between the respective steps, and a step flow rate. In addition, the pump A decides and sets a cooperation start timing and a cooperation end timing based on the flow rate set by the user (that is, sets various types of parameters necessary for the execution of infusion by the W method).

In step S403, the pump A accepts an instruction to cooperate with the other medical pump (the pump B in this case) to implement infusion by the W method.

Note that information concerning the drug selected in step S401 and a cooperation instruction accepted in step S403 are transmitted to the medical pump housing rack 200.

Likewise, the pump B accepts the selection of a drug to be administered in step S411.

In addition, in step S412, the pump B reads out a W method table stored in correspondence with the drug accepted in step S411, and sets a step count, the time interval between the respective steps, and a step flow rate. In addition, the pump B decides and sets a cooperation start timing and a cooperation end timing based on the flow rate set by the user (that is, sets various types of parameters necessary for the execution of infusion by the W method).

Note that information concerning the drug selected in step S411 and a cooperation instruction accepted in step S413 are transmitted to the medical pump housing rack 200.

The medical pump housing rack 200 determines in step S421, based on the information transmitted in steps S401 and S403 and steps S411 and S413, whether the pumps A and B can cooperate with each other.

If an instruction to make the pumps A and B cooperate with each other is selected in this manner, the medical pump housing rack 200 determines, based on the information transmitted from the pumps A and B, whether the pumps A and B can cooperate with each other. With this arrangement, even if the user has erroneously set a medical pump, which should not cooperate, as a cooperation target, the user is notified of the operation error (that is, it is possible to reduce operation errors by the user in various types of operations for infusion by the W method). Cooperation permission/inhibition determination processing will be described in detail later.

Upon determining, as a result of cooperation permission/inhibition determination processing in step S421, that the pumps can cooperate with each other, the medical pump housing rack 200 notifies the pumps A and B of the corresponding information (cooperation permission notification).

If the cooperation permission/inhibition determination processing result notified in step S421 indicates that the pumps can cooperate with each other, the pump A accepts the input of an infusion start instruction by the user (operation of issuing an infusion start instruction by the user becomes valid) in step S404.

Upon accepting the input of the instruction, the pump A notifies the medical pump housing rack 200 of the start of infusion, while starting infusion in step S405.

In step S422, upon receiving a notification indicating the start of infusion by the pump A from the pump A, the medical pump housing rack 200 notifies the pump B of the corresponding information.

Upon receiving the notification, the pump B validates the operation of issuing an infusion start instruction by the user. Subsequently, the pump B can accept an infusion start instruction until the cooperation start timing is reached.

In step S414, upon accepting the infusion start instruction input by the user, the pump B notifies the medical pump housing rack 200 of the corresponding information.

This makes the medical pump housing rack 200 recognize the completion of infusion preparation for the pump B in step S422.

The pump B as well as the pump A is configured to make the user input an infusion start instruction in consideration of the securement of safety for a patient. Assume that accepting an infusion start instruction from the pump A will make the pumps A and B automatically start infusion. With this arrangement, after an infusion start instruction for the pump A is input, the pump B starts infusion without making the user monitor the state of the pump B.

With the above arrangement, the medical pump housing rack 200 according to this embodiment can avoid such a situation.

In step S422, upon recognizing that infusion preparation for the pump B is complete, the medical pump housing rack 200 stands by until the cooperation start timing.

In this case, the cooperation start timing in this embodiment is one of the following timings:
- the timing at which the pump A completes infusion of a scheduled amount;
- the timing (T1 < T2) at which a calculated remaining time (T1) until a drug runs out at the current flow rate of the pump A becomes less than a preset time (T2); and
- the timing (T1 < T3) at which the calculated remaining time (T1) by which a drug runs out at the current flow rate of the pump A becomes less than a calculated arrival time (T3) at which the flow rate of the pump B arrives at a stable flow rate.

Note that if an instruction to change a flow rate setting for the pump A is input to change the flow rate setting during infusion by the pump A while the medical pump housing rack 200 stands by until the cooperation start timing, the pump A transmits the corresponding notification to the medical pump housing rack 200 (step S406). In this case, the medical pump housing rack 200 transmits the flow rate setting change notification received from the pump A to the pump B. Note however that the medical pump housing rack 200 may be configured to notify the pump B of a flow rate setting change notification every time it is issued. Alternatively, the medical pump housing rack 200 may be configured to notify the pump B of this information upon including it in a flow rate notification issued at the cooperation start timing.

When the cooperation start timing is reached and the pump A transmits a cooperation start instruction in step S407, the medical pump housing rack 200 recognizes, from the cooperation start instruction transmitted from the pump A, that cooperation has been established between the pumps A and B, and notifies the pumps A and B of the corresponding information (cooperation establishment notification) in step S422. These processes (cooperation establishment determination processing) in step S422 will be described in detail later.

In step S415, upon receiving a flow rate setting change notification from the medical pump housing rack 200, the pump B sets a step flow rate based on the flow rate setting after the change. In addition, upon receiving the notification indicating that cooperation has been established from the medical pump housing rack 200, the pump B starts infusion. Note that, as described above, the medical pump housing rack 200 may be configured to notify the pump B of a flow rate setting change every time the flow rate changes or to notify the pump B of a flow rate setting change upon including it in a flow rate notification issued at the cooperation start timing. If the medical pump housing rack 200 notifies the pump B of a flow rate setting change upon including it in a flow rate notification issued at the cooperation start timing, the pump B sets a step flow rate upon receiving the flow rate notification, and starts infusion. Note that these processes (infusion start processing) in the pump B will be described in detail later.

In this manner, the medical pump housing rack 200 is configured to determine that cooperation has been established between the pump A and the pump B and monitor the infusion states of the pumps A and B afterward. This makes it possible to avoid a situation in which the pump B automatically starts infusion in spite of the fact that infusion by the W method should not be maintained because some trouble has occurred in the pump B.

In addition, the arrangement of notifying the pump B via the medical pump housing rack 200 of the flow rate setting in the pump A in the process of infusion, which has been changed before the cooperation start timing, can reflect, in the pump B, the change in flow rate setting made by the user in the pump A.

### <5. Details of Operation at Time of Infusion by W Method>

The following is a detailed description about the cooperation permission/inhibition determination processing and cooperation establishment determination processing performed by the W method monitoring function of the medical pump housing rack 200 and infusion start processing by the pump B in operation at time of infusion by the W method.

### (1) Details of Cooperation

### Permission/Inhibition Determination Processing (Step S421)

A detailed procedure for cooperation permission/inhibition determination processing (step S421) by the medical pump housing rack 200 will be described first.

Fig. 5 is a flowchart showing a detailed procedure for cooperation permission/inhibition determination processing by the medical pump housing rack. Upon receiving cooperation instructions from the pumps A and B, the medical pump housing rack starts the cooperation permission/inhibition determination processing shown in Fig. 5.

In step S501, the medical pump housing rack identifies the housing positions of the pumps A and B which have transmitted the cooperation instructions. Note that the medical pump housing rack identifies the housing positions of the pumps A and B based on the port numbers corresponding to the reception of the cooperation instructions from the respective pumps.

In step S502, the medical pump housing rack determines, based on the housing positions identified in step S501, whether the pumps A and B which have transmitted the cooperation instructions are adjacent to each other. More specifically, if the respective port numbers are serial numbers, the medical pump housing rack determines that the pumps are adjacent to each other, and the process advances to step S503. If the respective port numbers are not serial numbers, the medical pump housing rack determines that the pumps are not adjacent to each other, and the process advances to step S506.

In step S506, the medical pump housing rack notifies the pumps A and B which have transmitted the cooperation instructions of inhibition of cooperation. As described above, a cooperation permission/inhibition condition is limited to that the housing positions of the pumps A and B should be adjacent to each other. This is because making medical pumps, of the plurality of medical pumps housed in the medical pump housing rack 200, which are located at distant positions cooperate with each other may cause the user to make an operation error.

In step S503, the medical pump housing rack identifies the drugs selected in the pumps A and B which have transmitted the cooperation instructions.
In step S504, the medical pump housing rack determines whether the drugs respectively identified in step S503 match.

If the medical pump housing rack determines in step S504 that the drugs do not match, the user may have selected a wrong drug on the pump A or B or may have erroneously operated a medical pump different from the medical pump to cooperate to input a cooperation instruction. In either case, the medical pump housing rack determines that the user has made an operator error, and notifies the pumps A and B which have transmitted the cooperation instructions of inhibition of cooperation.

If the medical pump housing rack determines in step S504 that the drugs match, the process advances to step S505 to notify the pumps A and B which have transmitted the cooperation instructions of permission of cooperation (cooperation permission notification).

As described above, the medical pump housing rack 200 according to this embodiment is configured to determine permission/inhibition of cooperation when the user inputs cooperation instructions on the pumps A and B and to issue a notification immediately upon determining inhibition of cooperation. This makes it possible to reduce operation errors made by the user.

The pump A which has received the notification of permission of cooperation from the medical pump housing rack 200 can accept an infusion start instruction. This allows the user to start infusion by the pump A.

(2) Details of Cooperation Establishment Determination Processing (Step S422)

A procedure for cooperation establishment determination processing by the medical pump housing rack 200 will be described next. Fig. 6 is a flowchart showing a detailed procedure for cooperation establishment determination processing by the medical pump housing rack. Upon receiving, from the pump A, a notification indicating that an infusion start instruction has been input, the medical pump housing rack starts the cooperation establishment determination processing shown in Fig. 6.

In step S601, the medical pump housing rack determines whether it has received a notification indicating that the pump B has input an infusion start instruction. If the medical pump housing rack determines in step S601 that it has received the notification, the process advances to step S602.

If the medical pump housing rack determines in step S601 that it has not received the notification, the process advances to step S606 to determine whether the cooperation start timing is reached (whether it has received a cooperation start instruction from the pump A). If the medical pump housing rack determines that the cooperation start timing is not reached, the process advances to step S602.

In step S602, the medical pump housing rack determines whether the user has changed the flow rate setting of the pump A during infusion. If the medical pump housing rack determines in step S602 that the user has changed the flow rate setting of the pump A during infusion, the process advances to step S603 to notify the pump B of the changed flow rate setting. The process then advances to step S604. Note that the pump B changes the step flow rate or the like based on the notification of the changed flow rate setting. However, the present invention is not limited to this. For example, although the changed flow rate setting is notified to the medical pump housing rack 200, the process may advance to step S604 without executing the processing in step S603. In this case, in step S605, the medical pump housing rack notifies the changed flow rate setting.

If the medical pump housing rack determines step S602 that the user has not changed the flow rate setting of the pump A during infusion, the process directly advances to step S604.

In step S604, the medical pump housing rack determines whether the cooperation start timing is reached (whether it has received a cooperation start instruction from the pump A). If the medical pump housing rack determines in step S604 that the cooperation start timing is not reached, the process returns to step S601. If the medical pump housing rack has not received a notification indicating that the pump B has input an infusion start instruction, the medical pump housing rack waits for an infusion start instruction from the pump B. If the medical pump housing rack has already received a notification indicating that the pump B has input an infusion start instruction, the medical pump housing rack stands by until the cooperation start timing is reached. If, however, the user has changed the flow rate setting of the pump A during infusion, the medical pump housing rack notifies the pump B of the change every time it occurs. However, the present invention is not limited to this. For example, every time the flow rate of the pump A is changed, the pump A notifies the medical pump housing rack 200 of the flow rate change. However, at this point of time, no information may be notified to the pump B, and the final flow rate of the pump A may be notified to the pump B.

If the medical pump housing rack determines in step S604 that the cooperation start timing is reached, the process advances to step S605 to notify the pumps A and B of the establishment of cooperation (cooperation establishment notification). This causes the pump B to start infusion.

If the medical pump housing rack determines in step S606 that the cooperation start timing is reached before receiving a notification indicating that a transmission start instruction has been input from the pump B, the process advances to step S607. In step S607, the medical pump housing rack notifies the pump B of a warning instruction. Upon being notified of the warning instruction, the pump B outputs a warning.

In step S608, the medical pump housing rack determines whether the user has stopped the pump B from outputting a warning. If the medical pump housing rack determines in step S608 that the user has stopped the pump B from outputting a warning, the process advances to step S605 to notify the pumps A and B of the establishment of cooperation (cooperation establishment notification).

Although not clearly shown in Fig. 6, if the pump A is removed from the medical pump housing rack 200 or infusion stops due to an abnormality before the cooperation start timing is reached, the medical pump housing rack notifies the pump B of the failure to establish cooperation with the pump B. In this case, the pump B does not automatically start infusion by the W method.

### (3) Details of Infusion Start Processing by Pump B (Step S415)

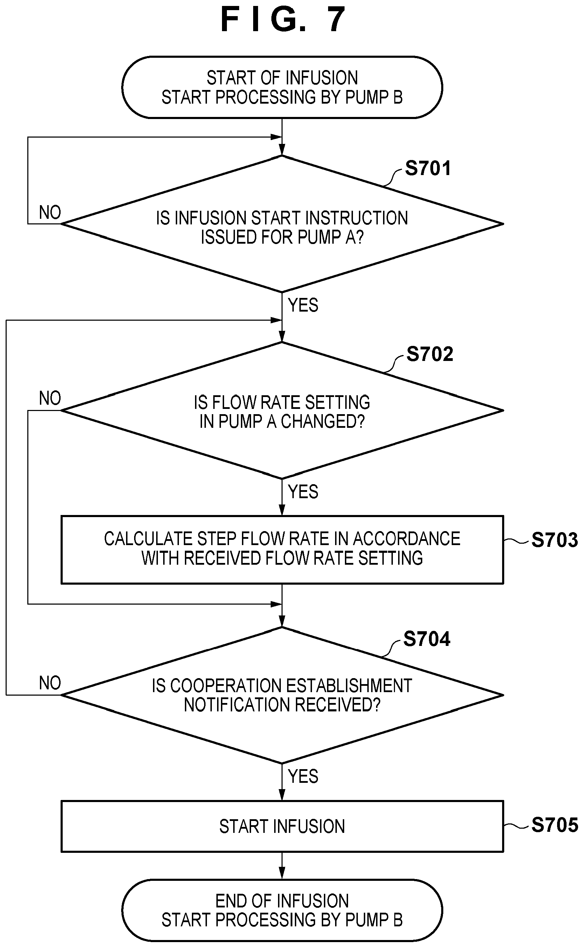

A procedure for infusion start processing by the pump B (step S415) will be described next. Fig. 7 is a flowchart showing a detailed procedure for infusion start processing by the pump B. Upon receiving a notification indicating that cooperation can be establishment from the medical pump housing rack 200, the pump B starts the infusion start processing shown in Fig. 7.

In step S701, the pump B determines whether the medical pump housing rack 200 has input an infusion start instruction to the pump A, and determines whether it has received a notification indicating that an infusion start instruction has been input to the pump A. If the pump B determines in step S701 that it has received, from the medical pump housing rack 200, a notification indicating that an infusion start instruction has been input to the pump A, the process advances to step S702. If the medical pump housing rack 200 is not configured to notify the pump B of a changed flow rate setting every time it is changed, the pump B does not receive a flow rate setting change notification concerning the pump A. For this reason, the process advances to step S704 without executing the step S702.

In step S702, the pump B determines whether the pump A has changed the flow rate setting during infusion and the medical pump housing rack 200 has notified the changed flow rate setting. If the pump B determines in step S702 that the medical pump housing rack 200 has not notified the changed flow rate setting, the process advances to step S704 (as described above, if step S702 is not executed, the process directly advances to step S704).

If the pump B determines in step S702 that the medical pump housing rack 200 has notified the changed flow rate setting, the process advances to step S703 to calculate and set a step flow rate or the like based on the notified flow rate setting (as described above, if step S702 is not executed, the process directly advances to step S704).

In step S704, the pump B determines whether it has received, from the medical pump housing rack 200, a notification indicating that cooperation has been established. If NO in step S704, the process returns to step S702 (note that if the medical pump housing rack 200 does not notify the pump B of a changed flow rate setting every time it is changed, the process returns to step S704).

If the pump B determines in step S704 that it has received a notification, the process advances to step S705 to start infusion (note that if the 200 does not notify the pump B of a changed flow rate setting every time it is changed, the pump B executes the processing in step S703 based on the received flow rate).

As is obvious from the above description, when monitoring infusion by the W method using a plurality of housed medical pumps,
- the medical pump housing rack according to this embodiment is configured to determine cooperation permission/inhibition with respect to the cooperation instructed by the user; and
- the medical pump housing rack is configured to define a cooperation establishment condition between the start of infusion by the pump A and the start of infusion by the pump B and cause the pump B to start infusion when the condition is satisfied.

### As described above, the medical pump housing rack is configured to monitor various operations performed by the user on medical pumps which perform infusion by the W method. According to this embodiment, therefore, it is possible to reduce operation errors made by the user.

The present invention is not limited to the above-described embodiments, and various changes and modifications can be made within the scope of the present invention. Therefore, to apprise the public of the scope of the present invention, the following claims are made.

This application claims priority of Japanese Patent Application No. 2010-218978, filed September 29, 2010.

## Claims

1. A medical pump housing rack (200) which includes a control unit (301) and a communication module (302), houses a plurality of medical pumps, and is communicatively connected to the plurality of medical pumps (221,222,223) via the communication module,
wherein the communication module (302) is configured to notify to the control unit (301) a received data from one of the medical pumps (221, 222, 223) via a corresponding one of the ports (211, 212, 213) of the medical pump housing rack (200) and a port number of the corresponding one of the ports (211, 212, 213), and
when the communication module (302) receives a pair of the port number and data from the control unit (301), the communication module (302) is configured to output the data from the control unit (301) to one of the ports (211, 212, 213) which corresponds to the port number;
and wherein the control unit (301) performs:
a cooperation permission/inhibition determination processing for determining whether two predetermined medical pumps (221,222) of the plurality of medical pumps (221,222,223) are configured to operate in cooperation with each other, based on information indicating housing positions at which the two medical pumps (221,222) are respectively housed and drug information respectively set for the two medical pumps(221,222); and
a cooperation permission notification transmission processing for, if it is determined in the cooperation permission/inhibition determination processing that the two medical pumps (221,222) are configured to operate in cooperation with each other when the two medical pumps are housed adjacent to each other and when the drug information respectively set for the two medical pumps (221, 222) match, transmitting, to the two medical pumps (221,222) which are communicatively connected together via the communication module (302), cooperation permission notifications for validating operation of issuing infusion start instructions for a drug to the two medical pumps(221,222).

2. The medical pump housing rack according to claim 1, **characterized in that** said cooperation permission/inhibition determination processing identifies medical pumps, of the plurality of housed medical pumps, to which a user issues an instruction to operate in cooperation with each other, and determines whether the identified medical pumps are configured to operate in cooperation with each other.

3. The medical pump housing rack according to claim 2, **characterized in that** the control unit further performs a cooperation start instruction transmission processing for transmitting a cooperation start instruction transmitted from a starting medical pump, of the two medical pumps to which cooperation permission notifications are transmitted by the cooperation permission notification transmission processing, which has started infusion first to a subsequent medical pump which starts infusion later, wherein when the cooperation start instruction transmission processing transmits the cooperation start instruction, the subsequent medical pump starts infusion.

4. The medical pump housing rack according to claim 3, **characterized in that** the control unit further performs a flow rate setting transmission processing for, upon receiving an instruction indicating that a flow rate setting during infusion from the starting medical pump has been changed, transmitting the changed flow rate setting to the subsequent medical pump before the cooperation start instruction is transmitted from the starting medical pump, wherein a flow rate setting in the subsequent medical pump is changed when said flow rate setting transmission means transmits the changed flow rate setting.

5. The medical pump housing rack according to claim 4, **characterized in that** the control unit further performs a warning transmission processing for transmitting a warning instruction to the subsequent medical pump if a user has not performed operation of issuing an infusion start instruction to the subsequent medical pump when the starting medical pump transmits the cooperation start instruction, wherein the cooperation start instruction transmission processing transmits the cooperation start instruction to the subsequent medical pump if the subsequent medical pump outputs a warning in response to transmission of a warning instruction from the warning transmission processing, and the user performs operation of stopping output of the warning.

6. A control method for a medical pump housing rack (200) which includes a control unit (301) and a communication module (302), and which houses a plurality of medical pumps and is communicatively connected to the medical pumps via the communication module,
wherein the method performs under controlling of the control unit (301):
the communication module (302) notifies to the control unit (301) a received data from one of the medical pumps (221, 222, 223) via a corresponding one of the ports (211, 212, 213) of the medical pump housing rack (200) and a port number of the corresponding one of the ports (211, 212, 213), and
when the communication module (302) receives a pair of the port number and data from the control unit (301), the communication module (302) outputs the data from the control unit (301) to one of the ports (211, 212, 213) which corresponds to the port number;
the control unit (301) performing a cooperation permission/inhibition determination step of determining whether two predetermined medical pumps of the plurality of housed medical pumps are configured to operate in cooperation with each other, based on information indicating housing positions at which the two medical pumps are respectively housed and drug information respectively set for the two medical pumps; and
the control unit further performing a cooperation permission notification transmission step of, if it is determined in the cooperation permission/inhibition determination step that the two medical pumps are configured to operate in cooperation with each other when the two medical pumps are housed adjacent to each other and when the drug information respectivery set for the two medical pumps (221, 222) match, transmitting, to the two medical pumps (221, 222) which are communicatively connected together via the communication module (302), cooperation permission notifications for validating operation of issuing infusion start instructions for a drug to the two medical pumps.

7. A computer program for causing a computer, when said program is executed on said computer, to execute each step in a control method as defined in claim 6.

## Patentansprüche

1. Gehäusegestell bzw. -träger (200) für medizinische Pumpen, das bzw. der eine Steuer- bzw. Regeleinheit (301) und ein Kommunikationsmodul (302) enthält, eine Mehrzahl medizinischer Pumpen aufnimmt bzw. unterbringt und über das Kommunikationsmodul kommunikativ mit der Mehrzahl medizinischer Pumpen (221, 222, 223) verbunden ist,
wobei das Kommunikationsmodul (302) konfiguriert ist, die Steuer- bzw. Regeleinheit (301) über empfangene Daten von einer der medizinischen Pumpen (221, 222, 223) über einen entsprechenden der Ports (211, 212, 213) des Gehäusegestells (200) der medizinischen Pumpe und eine Portnummer des entsprechenden der Ports (211, 212, 213) zu benachrichtigen, und
wenn das Kommunikationsmodul (302) ein Paar von Portnummer und Daten von der Steuer- bzw. Regeleinheit (301) empfängt, das Kommunikationsmodul (302) konfiguriert ist, die Daten von der Steuer- bzw. Regeleinheit (301) an einen der Ports (211, 212, 213) auszugeben, welcher der Portnummer entspricht;
und wobei die Steuer- bzw. Regeleinheit (301) durchführt:
eine Kooperationserlaubnis/-verhinderung-Bestimmungsverarbeitung zum Bestimmen, ob zwei vorbestimmte medizinische Pumpen (221, 222) der Mehrzahl medizinischer Pumpen (221, 222, 223) konfiguriert sind, in Kooperation miteinander zu arbeiten, und zwar basierend auf Informationen, die Gehäusepositionen angeben, an denen die beiden medizinischen Pumpen (221, 222) jeweils untergebracht sind, und Medikamenteninformationen, die jeweils für die beiden medizinischen Pumpen (221, 222) festgelegt sind; und
eine Kooperationserlaubnis-Benachrichtigungsübertragungsverarbeitung, falls in der Kooperationserlaubnis/-verhinderung-Bestimmungsverarbeitung bestimmt wird, dass die beiden medizinischen Pumpen (221, 222) konfiguriert sind, in Kooperation miteinander zu arbeiten, wenn die beiden medizinischen Pumpen angrenzend bzw. benachbart zueinander untergebracht sind und wenn die jeweils für die beiden medizinischen Pumpen (221, 222) festgelegten Medikamenteninformationen übereinstimmen, zum Übertragen an die beiden medizinischen Pumpen (221, 222), die über das Kommunikationsmodul (302) kommunikativ miteinander verbunden sind, von Kooperationserlaubnisbenachrichtigungen zum Validieren des Vorgangs des Erteilens von Infusionsstartanweisungen für ein Medikament an die beiden medizinischen Pumpen (221, 222).

2. Gehäusegestell für medizinische Pumpen nach Anspruch 1, **dadurch gekennzeichnet, dass** die Kooperationserlaubnis/-verhinderung-Bestimmungsverarbeitung medizinische Pumpen der Mehrzahl untergebrachter medizinischer Pumpen identifiziert, denen ein Benutzer eine Anweisung zum Arbeiten in Kooperation miteinander erteilt, und bestimmt, ob die identifizierten medizinischen Pumpen konfiguriert sind, in Kooperation miteinander zu arbeiten.

3. Gehäusegestell für medizinische Pumpen nach Anspruch 2, **dadurch gekennzeichnet, dass** die Steuer- bzw. Regeleinheit ferner eine Kooperationsstartanweisung-Übertragungsverarbeitung zum Übertragen einer Kooperationsstartanweisung, die von einer beginnenden medizinischen Pumpe der beiden medizinischen Pumpen übertragen wird, an die Kooperationserlaubnisbenachrichtigungen durch die Kooperationserlaubnis-Benachrichtigungsübertragungsverarbeitung übertragen werden, die zunächst die Infusion begonnen hat, an eine nachfolgende medizinische Pumpe durchführt, welche die Infusion später beginnt, wobei wenn die Kooperationsstartanweisung-Übertragungsverarbeitung die Kooperationsstartanweisung überträgt, die nachfolgende medizinische Pumpe die Infusion beginnt.

4. Gehäusegestell für medizinische Pumpen nach Anspruch 3, **dadurch gekennzeichnet, dass** die Steuer- bzw. Regeleinheit ferner eine Flussrateneinstellübertragungsverarbeitung durchführt, um bei Empfang einer Anweisung, die angibt, dass eine Flussrateneinstellung während der Infusion von der beginnenden medizinischen Pumpe geändert wurde, die geänderte Flussrateneinstellung an die nachfolgende medizinische Pumpe zu übertragen, bevor die Kooperationsstartanweisung von der beginnenden medizinischen Pumpe übertragen wird, wobei eine Flussrateneinstellung bei der nachfolgenden medizinischen Pumpe geändert wird, wenn das Flussrateneinstellübertragungsmittel die geänderte Flussrateneinstellung überträgt.

5. Gehäusegestell für medizinische Pumpen nach Anspruch 4, **dadurch gekennzeichnet, dass** die Steuer- bzw. Regeleinheit ferner eine Warnübertragungsverarbeitung zum Übertragen einer Warnanweisung an die nachfolgende medizinische Pumpe durchführt, wenn ein Benutzer den Vorgang des Erteilens von Infusionsstartanweisungen an die nachfolgende medizinische Pumpe nicht durchgeführt hat, wenn die beginnende medizinische Pumpe die Kooperationsstartanweisung überträgt, wobei die Kooperationsstartanweisung-Übertragungsverarbeitung die Kooperationsstartanweisung an die nachfolgende medizinische Pumpe überträgt, wenn die nachfolgende medizinische Pumpe eine Warnung als Antwort auf eine Übertragung einer Warnanweisung von der Warnübertragungsverarbeitung ausgibt, und der Benutzer einen Vorgang des Stoppens der Ausgabe der Warnung durchführt.

6. Steuerungs- bzw. Regelungsverfahren für ein Gehäusegestell bzw. -träger (200) für medizinische Pumpen, das eine Steuer- bzw. Regeleinheit (301) und ein Kommunikationsmodul (302) enthält und das eine Mehrzahl medizinischer Pumpen aufnimmt bzw. unterbringt und über das Kommunikationsmodul kommunikativ mit der Mehrzahl medizinischer Pumpen (221, 222, 223) verbunden ist, wobei das Verfahren unter der Steuerung bzw. Regelung der Steuer- bzw. Regeleinheit (301) durchführt:
das Kommunikationsmodul (302) benachrichtigt die Steuer- bzw. Regeleinheit (301) über empfangene Daten von einer der medizinischen Pumpen (221, 222, 223) über einen entsprechenden der Ports (211, 212, 213) des Gehäusegestells (200) der medizinischen Pumpe und eine Portnummer des entsprechenden der Ports (211, 212, 213), und
wenn das Kommunikationsmodul (302) ein Paar von Portnummer und Daten von der Steuer- bzw. Regeleinheit (301) empfängt, das Kommunikationsmodul (302) die Daten von der Steuer- bzw. Regeleinheit (301) an einen der Ports (211, 212, 213) ausgibt, welcher der Portnummer entspricht;
wobei die Steuer- bzw. Regeleinheit (301) einen Kooperationserlaubnis/- verhinderung-Bestimmungsschritt des Bestimmens durchführt, ob zwei vorbestimmte medizinische Pumpen der Mehrzahl untergebrachter medizinischer Pumpen konfiguriert sind, in Kooperation miteinander zu arbeiten, und zwar basierend auf Informationen, die Gehäusepositionen angeben, an denen die beiden medizinischen Pumpen jeweils untergebracht sind, und Medikamenteninformationen, die jeweils für die beiden medizinischen Pumpen festgelegt sind; und
wobei die Steuer- bzw. Regeleinheit ferner einen Kooperationserlaubnis-Benachrichtigungsübertragungsschritt, falls in den Kooperationserlaubnis/- verhinderung-Bestimmungsschritt bestimmt wird, dass die beiden medizinischen Pumpen konfiguriert sind, in Kooperation miteinander zu arbeiten, wenn die beiden medizinischen Pumpen angrenzend bzw. benachbart zueinander untergebracht sind und wenn die jeweils für die beiden medizinischen Pumpen (221, 222) festgelegten Medikamenteninformationen übereinstimmen, des Übertragens an die beiden medizinischen Pumpen (221, 222), die über das Kommunikationsmodul (302) kommunikativ miteinander verbunden sind, von Kooperationserlaubnisbenachrichtigungen zum Validieren des Vorgangs des Erteilens von Infusionsstartanweisungen für ein Medikament an die beiden medizinischen Pumpen durchführt.

7. Computerprogramm, das einen Computer veranlasst, wenn das Programm auf dem Computer ausgeführt wird, jeden Schritt in einem Steuerungs- bzw. Regelungsverfahren nach Anspruch 6 auszuführen.

## Revendications

1. Support de réception de pompe médicale (200) qui comporte une unité de commande (301) et un module de communication (302), reçoit une pluralité de pompes médicales, et est couplé de manière à pouvoir communiquer à la pluralité des pompes médicales (221, 222, 223) par l'intermédiaire du module de communication,
dans lequel
le module de communication (302) est configuré de manière à notifier à l'unité de commande (301) des données reçues à partir de l'une des pompes médicales (221, 222, 223) par l'intermédiaire de l'un correspondant des ports (211, 212, 213) du support de réception de pompe médicale (200) et d'un numéro de port de l'un correspondant des ports (211, 212, 213), et
lorsque le module de communication (302) reçoit une paire composée du numéro de port et des données à partir de l'unité de commande (301), le module de communication (302) est configuré de manière à délivrer les données à partir de l'unité de commande (301) vers l'un des ports (211, 212, 213) qui correspond au numéro de port ;
et dans lequel l'unité de commande (301) exécute :
un traitement de détermination de permission/inhibition de coopération destiné à déterminer si les deux pompes médicales (221, 222) prédéterminées de la pluralité de pompes médicales (221, 222, 223) sont configurées de manière à fonctionner en coopérant l'une avec l'autre, sur la base d'informations indiquant les positions de réception auxquelles les deux pompes médicales (221, 222) sont respectivement agencées et les informations de médicament définies respectivement pour les deux pompes médicales (221, 222) ; et
un traitement de transmission de notification de permission de coopération destiné, s'il est déterminé dans le traitement de détermination de permission/inhibition de coopération que les deux pompes médicales (221, 222) sont configurées de manière à fonctionner en coopérant l'une avec l'autre lorsque les deux pompes médicales sont agencées de manière adjacente l'une par rapport à l'autre et lorsque les informations de médicament définies respectivement pour les deux pompes médicales (221, 222) correspondent, à transmettre, aux deux pompes médicales (221, 222) qui sont couplées de manière à pouvoir communiquer entre elles par l'intermédiaire du module de communication (302), des notifications de permission de coopération afin de valider une opération de délivrance d'instructions de début d'administration d'un médicament vers les deux pompes médicales (221, 222).

2. Support de réception de pompe médicale selon la revendication 1, **caractérisé en ce que** ledit traitement de détermination de permission/inhibition de coopération identifie des pompes médicales, parmi la pluralité de pompes médicales agencées, auxquelles un utilisateur délivre une instruction destinée à les faire fonctionner en coopération l'une avec l'autre, et détermine si les pompes médicales identifiées sont configurées de manière à fonctionner en coopération l'une avec l'autre.

3. Support de réception de pompe médicale selon la revendication 2, **caractérisé en ce que** l'unité de commande exécute, en outre, un traitement de transmission d'instruction de début de coopération destiné à transmettre une instruction de début de coopération transmise à partir d'une pompe médicale initiale, parmi les deux pompes médicales auxquelles les notifications de permission de coopération sont transmises par le traitement de transmission de notification de permission de coopération, qui a démarré l'administration en premier vers la pompe médicale suivante qui commence l'administration ultérieurement,
dans lequel lorsque le traitement de transmission d'instruction de début de coopération transmet l'instruction de début de coopération, la pompe médicale suivante initie l'administration.

4. Support de réception de pompe médicale selon la revendication 3, **caractérisé en ce que** l'unité de commande exécute, en outre, un traitement de transmission de réglage de débit destiné, lors de la réception d'une instruction indiquant qu'un réglage de débit au cours de l'administration à partir de la pompe médicale initiale a été modifié, à transmettre le réglage de débit modifié à la pompe médicale suivante avant que l'instruction de début de coopération soit transmise à partir de la pompe médicale initiale, dans lequel le réglage de débit sur la pompe médicale suivante est modifié lorsque ledit moyen de transmission de réglage de débit transmet le réglage de débit modifié.

5. Support de réception de pompe médicale selon la revendication 4, **caractérisé en ce que** l'unité de commande exécute, en outre, un traitement de transmission d'alerte destiné à transmettre une instruction d'alerte à la pompe médicale suivante si un utilisateur n'a pas exécuté une opération de diffusion d'une instruction de début d'administration à la pompe médicale suivante lorsque la pompe médicale initiale transmet l'instruction de début de coopération, dans lequel le traitement de transmission d'instruction de début de coopération transmet l'instruction de début de coopération à la pompe médicale suivante si la pompe médicale suivante délivre une alerte en réponse à la transmission d'une instruction d'alerte à partir du traitement de transmission d'alerte, et l'utilisateur exécute l'opération d'arrêt de production de l'alerte.

6. Procédé de commande d'un support de réception de pompe médicale (200) qui comporte une unité de commande (301) et un module de communication (302), et qui contient une pluralité de pompes médicales et est couplé de manière à pouvoir communiquer aux pompes médicales par l'intermédiaire du module de communication,
dans lequel le procédé s'exécute sous la commande de l'unité de commande (301) :
le module de communication (302) notifie à l'unité de commande (301) les données reçues à partir de l'une des pompes médicales (221, 222, 223) par l'intermédiaire de l'un correspondant des ports (211, 212, 213) du support de réception de pompe médicale (200) et d'un numéro de port de celui correspondant des ports (211, 212, 213), et
lorsque le module de communication (302) reçoit une paire composée du numéro de port et des données à partir de l'unité de commande (301), le module de communication (302) délivre les données à partir de l'unité de commande (301) à l'un des ports (211, 212, 213) qui correspond au numéro de port ;
l'unité de commande (301) exécute une étape de détermination de permission/inhibition de coopération destinée à déterminer si deux pompes médicales prédéterminées de la pluralité de pompes médicales intégrées sont configurées de manière à fonctionner en coopération l'une avec l'autre, sur la base d'informations indiquant des positions de réception auxquelles les deux pompes médicales sont respectivement reçues et d'informations de médicament définies respectivement pour les deux pompes médicales ; et
l'unité commande exécutant, en outre une étape de transmission de notification de permission de coopération destinée, s'il est déterminé dans l'étape de détermination de permission/inhibition de coopération que les deux pompes médicales sont configurées de manière à fonctionner en coopérant l'une avec l'autre lorsque les deux pompes médicales sont agencées de manière adjacente l'une par rapport à l'autre et lorsque les informations de médicament définies respectivement pour les deux pompes médicales (221, 222) correspondent, à transmettre, aux deux pompes médicales (221, 222) qui sont couplées de manière à pouvoir communiquer entre elles par l'intermédiaire du module de communication (302), des notifications de permission de coopération, destinées à valider l'opération de délivrance d'instructions de début d'administration d'un médicament aux deux pompes médicales.

7. Programme informatique destiné à amener un ordinateur, lorsque ledit programme est exécuté sur ledit ordinateur, à exécuter chaque étape d'un procédé de commande selon la revendication 6.
